# EUROPEAN PATENT SPECIFICATION

(11) **EP 4 077 255 B1**
(45) Date of publication and mention of the grant of the patent: **31.01.2024**
(21) Application number: 20821235.7
(22) Date of filing: 14.12.2020
(51) Int. Cl.: C07C 37/07, C07C 39/08

(54) **PROCESS FOR THE PREPARATON OF HYDROQUINONES**
VERFAHREN ZUR HERSTELLUNG VON HYDROCHINONEN
PROCÉDÉ DE PRÉPARATION D'HYDROQUINONES

(30) Priority: 19.12.2019 EP 19217785
(43) Date of publication of application: 26.10.2022
(73) Proprietor: DSM IP Assets B.V., 6411 TE Heerlen (NL)
(72) Inventor: BONRATH, Werner, 4303 Kaiseraugst (CH); NETSCHER, Thomas, 4303 Kaiseraugst (CH); MEDLOCK, Jonathan Alan, 4303 Kaiseraugst (CH)
(74) Representative: dsm-firmenich IP
(86) International application number: PCT/EP2020/085940
(87) International publication number: WO 2021/122436

(56) References cited:
- EP-A1- 0 264 823
- DE-A1- 2 250 066
- BENJAMIN SCHÄFFNER ET AL: "Organic Carbonates as Solvents in Synthesis and Catalysis", CHEMICAL REVIEWS, vol. 110, no. 8, 11 August 2010 (2010-08-11), pages 4554-4581, XP055618498, US ISSN: 0009-2665, DOI: 10.1021/cr900393d

## Description

### Technical Field

The present invention relates to the preparation of hydroquinones from the respective quinones.

### Background of the invention

The preparation of hydroquinones from quinones is known to the person skilled in the art. Hydroquinones are important intermediates. Particularly, methyl- or dimethyl- or trimethyl-hydroquinones are known as intermediates for tocopherol synthesis. Particularly important is the reduction of 2,3,5-trimethylquinone (TMQ) as 2,3,5-trimethylhydroquinone (TMHQ) is a well-known intermediate for α-tocopherol.

The reduction of TMHQ by catalytic hydrogenation in presence of an organic solvent is well known. For example, DE 683 908 describes methanol and ethanol as solvent. These solvents, however, lead to autoxidation of TMHQ and undesired coloration and, hence, are considered as being unsuitable solvents for this reaction. Using higher aliphatic alcohols or unpolar solvents such as toluene, TMHQ, the product of hydrogenation, is precipitated and leads to incrustation which increase cost for maintenance of the reaction equipment as well as for the regeneration of the hydrogenation catalyst. DE 1 940 386 solves this problem by using an aliphatic alcohol with 3 to 5 carbon atoms as solvent and using a temperature of 60 - 180°C. EP 0 264 823 A1 propose other solvents such as ketones, organic acids, organic acid esters, ethers, cyclic ethers, and lactones as solvents for the hydrogenation of solvents. However, these solvents have deficiencies in view of environmental aspects, chemical stability towards acids and/or bases and toxicology or fire hazards. DE 22 50 066 A1 discloses the reduction of 2,3,5-trimethylbenzoquone with hydrogen in the presence of a hydrogenation catalyst in a carbonic acid ester with 2 to 12 carbon atoms.

Cyclic alkyl carbonates (=alkylene carbonates) are, somehow, still unusual solvents for chemical reactions, the more and more, however, these solvents are used due to excellent ecotoxicity and positive fire and working hazard properties. Finally, alkylene carbonates are attractive in view of cost.

However, as discussed in US 2003/0060383 A1, an unfortunate property of alkylene carbonates which is well-known to those skilled in the art, is that these compounds are prone to hydrolysis. The hydrolysis leads to formation of CO₂ and the respective alkanediol. Furthermore, it is also known to the person skilled in the art, for example from P. Ziosi et al., Catal. Sci. Technol. 2014, 4, 4386-4395, that phenol and alkylene carbonates form different alkanediol ethers of the phenols.

### Summary of the invention

Therefore, the problem to be solved by the present invention is to reduce quinones of the formula (III) to hydroquinones of the formula (I) in a solvent which assures that the quinones of the formula (III) as well as the hydroquinones of the formula (I) remain dissolved and in which the formation of side products is mostly avoided.

The process according to claim 1 is surprisingly suited to solve this problem. It particularly has been found that no precipitation of the hydroquinone has been formed and that the alkylene carbonates of the formula (X) used as solvents does not decompose under the conditions of the reduction. Contrary to the expectation, no indication of any degradation of the solvent, such as formation of CO₂ or an increased amount of alkanediols or the respective alkanediol ether derivatives of the hydroquinones has been found.

Further aspects of the invention are subject of further independent claims. Particularly preferred embodiments are subject of dependent claims.

### Detailed description of the invention

In a first aspect the present invention relates to a process for the manufacturing the compound of the formula (I) by reduction of the compound of the formula (III) 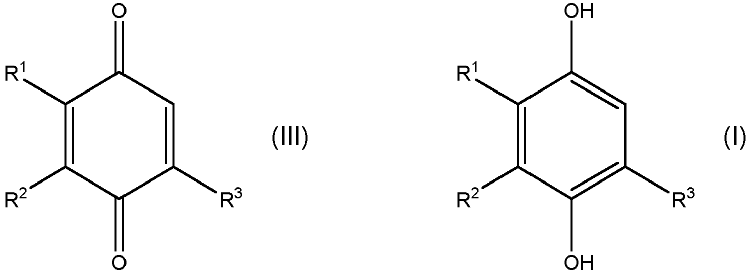
wherein R¹, R² and R³ independently represent either H or a C₁₋₄-alkyl group with the proviso that at least one of the residues R¹, R² and R³ is different from H;
characterized in that the reduction is performed by a reducing agent in at least one solvent of the formula (X) 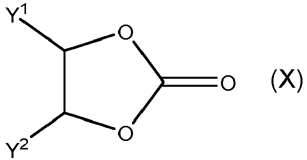 wherein Y¹ and Y² represent independently from each other either H or a methyl or ethyl group;
or in a two-phase solvent mixture comprising at least one solvent **(A)** being of a solvent of the formula (X) and at least one solvent **(B)** which is not miscible with solvent of the formula (X) at room temperature in a volume ratio of (**A**)/(**B**) of between 1/50 and 50/1.

For sake of clarity, some terms as been used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

A "C_{x-y}-alkane" accordingly corresponds to a linear or branched alkane comprising x to y carbon atoms.

In case identical labels for symbols or groups are present in several formulae, in the present document, the definition of said group or symbol made in the context of one specific formula applies also to other formulae which comprises the same said label.

The term "independently from each other" in this document means, in the context of substituents, moieties, or groups, that identically designated substituents, moieties, or groups can occur simultaneously with a different meaning in the same molecule.

The term "inert", as used in this document in describing a material, means that under the conditions of the reaction said material does not undergo any chemical reaction.

### Compound of the formula (III)

The compound of the formula (III) are well known to the person skilled in the art.

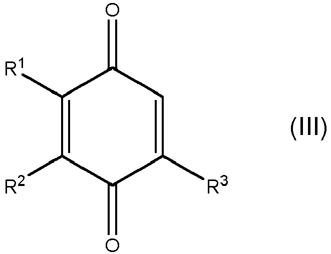

R¹, R² and R³ independently represent preferably either H or a methyl group.

Preferred are the following combinations of R¹, R² and R³:

R¹ = R² = R³ = CH₃

or

R¹ = R³ = CH₃, R² = H

or

R¹ = R² = CH₃, R³ = H,

or

R¹ = CH₃, R² = R³ = H,

Most preferred is the combination R¹ = R² = R³ = CH₃.

In other words, the most preferred compound of the formula (III) is 2,3,5-trimethylbenzoquinone (=2,3,5-trimethylcyclohexa-2,5-diene-1,4-dione) and the most preferred compound of the formula (I) is 2,3,5-trimethylhydroquinone (=2,3,5-trimethylbenzene-1,4-diol).

### Reducing agent

The compound of the formula (III) is reduced in the above process to the compound of the formula (I) by a reducing agent.

In one preferred embodiment of the invention the reducing agent is molecular hydrogen. In other words, in one embodiment the compound of the formula (III) is hydrogenated by molecular hydrogen in the presence of a metal catalyst.

The metal of the metal catalyst is preferably selected from the group consisting of Ni, Ir, Pd, Pt, Rh and Ru, particularly Pd or Pt, particularly a heterogeneous metal catalyst on a carrier or a support material.

The metal catalyst can be a catalyst comprising more than one of the mentioned metals.

It is preferred that the metal of the metal catalyst is palladium.

The metal catalyst is preferably a heterogeneous metal catalyst on a carrier or a support material.

Such carrier material is particularly a solid material having a high surface area, to which the metal is affixed. The support may be inert or participate in the catalytic reactions.

Typical supports/carrier material include various kinds of carbon or an oxide of Si, Al, Ce, Ti or Zr, particularly of Al or Si, such as alumina or silica. The preferred support/carrier material is carbon.

In case the support is Cer, the preferred oxide is CeO₂. Preferably, the oxide of Al is Al₂O₃ and AlO(OH). Particularly preferred is Boehmite.

The surface area of the carrier is preferably in the range of 800 to 1500 m²/g, particularly of 1000 to 1200 m²/g.

The heterogeneous metal catalyst may also be affixed or immobilized on a surface of a larger object typically in form of a structured packing element which might be a part of the reactor in which the reduction takes place or an element which is inserted into said reactor.

The support material is, hence, preferably a structured packing element. This structured packing element may be a dumped packing, a knit, an open-celled foam structure, preferably made of plastic, for example polyurethane or melamine resin, or ceramic, or a structured packing element, as already known in principle, i.e. by its geometric shape, from distillation and extraction technology. However, for the purposes of the present invention, structured packings in principle have a substantially smaller hydraulic diameter, frequently by a factor of from 2 to 10, than comparable internals in the field of distillation and extraction technology. Useful structured packing elements are in particular metal fabric packings and wire fabric packings, for example of the design Montz A3, Sulzer BX, DX and EX. Instead of metal fabric packings, it is also possible to use structured packings made of other woven, knitted or felted materials. Further useful structured packings are of flat or corrugated sheets, preferably without perforation, or other relatively large orifices, for example corresponding to the designs Montz BI or Sulzer Mellapak. The structured packings made of expanded metal are also advantageous, for example structured packings of the type Montz BSH.

It is preferred that the metal catalyst is a palladium catalyst, particularly a palladium on carbon catalyst (Pd/C).

The catalytic metal loading (weight ratio metal/catalyst, i.e. the weight metal / weight (metal+carrier)) is typically between 1 to 20%, preferably between 4 and 11%, more preferably between 4 and 6% by weight. A very preferred heterogeneous metal catalyst is palladium on carbon catalyst (Pd/C) of which 5 % by weight is palladium (i.e. loading = 5%).

In another preferred embodiment of the invention the reducing agent is a transfer hydrogenation agent. In other words, in another embodiment the compound of the formula (III) is transfer hydrogenated by a transfer hydrogenation agent to yield the compound of the formula (I). Said transfer hydrogenation agent is preferably formic acid and/or a formic acid salt.

In an even further preferred embodiment of the invention the reducing agent is a dithionite salt. The reduction can be achieved with sodium dithionite in water according to the method as disclosed by K. Sato, Y. Fujima, A. Yamada Bull. Chem. Soc. Jap. 1968, 41, 442-444.

Hence, preferably the reducing agent is either molecular hydrogen or a transfer hydrogenation agent or a dithionite salt.

### Solvent of the formula (X)

The reduction of compound of the formula (III) to the compound of the formula (I) is performed in at least one solvent of the formula (X) 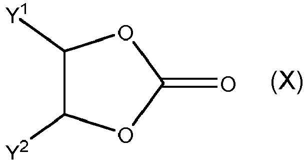
wherein Y¹ and Y² represent independently from each other either H or a methyl or ethyl group;
or in a two-phase solvent mixture comprising at least one solvent (**A**) being of a solvent of the formula (X) and at least one solvent (**B**) which is not miscible with solvent of the formula (X) at room temperature in a volume ratio of (**A**)/(**B**) of between 1/50 and 50/1.

The substituent Y¹ is preferably H.

In a preferred embodiment, Y¹ = Y² = H. Hence, ethylene carbonate is a preferred solvent of the formula (X).

In a further preferred embodiment, Y¹ = H and Y² = CH₃. Hence, propylene carbonate is another preferred solvent of the formula (X).

In a further preferred embodiment, Y¹ = H and Y² = CH₂CH₃. Hence, butylene carbonate is another preferred solvent of the formula (X).

Ethylene carbonate and propylene carbonate are the most preferred solvents of the formula (X).

In a very preferred embodiment, the reduction is performed in at least two solvents of the formula (X), particularly a binary or ternary mixture of ethylene carbonate and/or propylene carbonate and/or butylene carbonate, most preferably a binary mixture of ethylene carbonate and propylene carbonate. It is preferred that the ratio of ethylene carbonate to propylene carbonate is between 20:80 to 80:20, particularly 25:75 to 75:25.

Preferably, the solvent of the formula is a carbonate solvent as commercialized by Huntsman under the trademark Jeffsol^{®}, particularly the blends of ethylene carbonate with propylene carbonate Jeffsol^{®} EC-75, Jeffsol^{®} EC-50 and Jeffsol^{®} EC-25.

In a further embodiment, the reduction is performed in a two-phase solvent mixture comprising at least one solvent (**A**) being of a solvent of the formula (X) and at least one solvent (**B**) which is not miscible with solvent of the formula (X) at room temperature in a volume ratio of (**A**)/(**B**) of between 1/50 and 50/1.

The solvent (**B**) which is not miscible with the solvent (**A**) of the formula (X) is preferably a hydrocarbon, more preferably an alkane, particularly a C₅₋₁₆-alkane, most particularly a C₆₋₈-alkane. Most preferably the solvent (B) is either hexane or heptane.

It has been shown that the above process allows to reduce the compound of the compound of the formula (III) in an efficient way to the compound of the formula (I). The use of the solvent of formula (X) is very advantageous as the starting material, i.e. the compound of formula (III) as well as the compound of the formula (I) are well soluble and no precipitation occurs. The good solubility also allows that the process can be performed at low temperatures, particularly at temperatures below 70 °C, more preferably at temperatures between the melting point of the solvent of the formula (X) and 60°C.

The solubility of hydrogen in the solvent of formula (X) is very high, allowing a good yield and selectivity.

Furthermore, it has been observed that in said process the solvent of the formula is very stable under the reaction conditions. It has been surprisingly not observed that the solvent is decomposed into the respective diol and CO₂. Furthermore, no formation of ethers between the respective diol and the phenol of the formula (I) have been observed.

After the end of the reaction the catalyst can be easily removed from the reaction mixture. Particularly useful for this separation are either filtration or extraction.

The process is also very advantageous in that on the one hand the compound of the formula (III) can be prepared in the solvent of the formula (X) and the condensation of the compound of the formula (I) with phytol or isophytol to yield tocopherol on the other hand can be performed in the solvent of the formula (X). This is particularly advantageous as no change of solvent needs to be made during the whole process starting from (poly) alkylphenol to tocopherol.

Alkylene carbonates are very attractive solvents due to their excellent ecotoxicity and positive fire and working hazard properties as well as in view of cost.

Therefore, in a further embodiment, the invention relates to the use of ethylene carbonate and/or propylene carbonate and/or butylene carbonate as a solvent for the reduction of 2,3,5-trimethyl-1,4-benzoquinone to 2,3,5-trimethylhydroquinone.

### Examples

The present invention is further illustrated by the following experiments.

### Reduction of TMQ

30.24 g (200 mmol) of 2,3,5-trimethylquinone (TMQ) dissolved in 122 g of the warm solvent as given in table 1 have been added to a 350 ml 4-neck glass flask. 0.048 mol % (0.0934 mmol) Pd/C hydrogenation catalyst (5% metal loading) have been added. After inertisation with nitrogen (3 rinsing/evacuation cycles) molecular hydrogen has been added and heated to 80°C under stirring. After 30 minutes no hydrogen was consumed anymore. The reaction was stirred for another 30 minutes at this temperature.

After removal of the catalyst, a clear solution **A** was obtained without any precipitation. From this solution the formed product, i.e. 2,3,5-trimethylhydroquinone (TMHQ), can be isolated. Such isolation by extraction show a high yield

**Table 1: Reduction of TMQ to TMHQ**

| | ***2*** | ***3*** |
|---|---|---|
| Solvent | Propylene carbonate | Ethylene carbonate |
| Yield [%] | 94.5 | 96.0 |

## Claims

1. A process for the manufacturing the compound of the formula (I) by reduction of the compound of the formula (III) 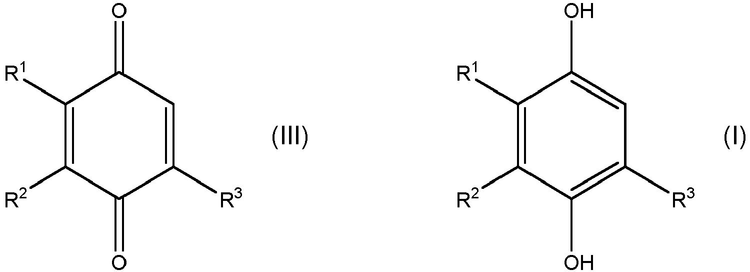
wherein R¹, R² and R³ independently represent either H or a C₁₋₄-alkyl group with the proviso that at least one of the residues R¹, R² and R³ is different from H;
**characterized in that** the reduction is performed by a reducing agent in at least one solvent of the formula (X) 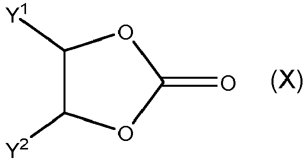 wherein Y¹ and Y² represent independently from each other either H or a methyl or ethyl group;
or in a two-phase solvent mixture comprising at least one solvent (**A**) being of a solvent of the formula (X) and at least one solvent (**B**) which is not miscible with solvent of the formula (X) at room temperature in a volume ratio of (**A**)/(**B**) of between 1/50 and 50/1.

2. The process according to claim 1, **characterized in that** Y¹ = Y² = H.

3. The process according to claim 1, **characterized in that** Y¹ = H and Y² = CH₃.

4. The process according to anyone of the preceding claims, **characterized in that** the reduction is performed in a solvent mixture of a solvent (**A**) of the formula (X) and a solvent (**B**) being hydrocarbon, preferably an alkane, more preferably a C₅₋₁₆-alkane, particularly a C₆₋₈-alkane.

5. The process according to anyone of the preceding claims, **characterized in that** the reduction is performed in a solvent mixture of at least two solvents of the formula (X), particularly in a mixture of ethylene carbonate and propylene carbonate.

6. The process according to anyone of the preceding claims **characterized in that** the reduction is performed using a reducing agent which is either molecular hydrogen or a transfer hydrogenation agent or a dithionite salt.

7. The process according to anyone of the preceding claims **characterized in that** the reduction is performed using a reducing agent which is molecular hydrogen in the presence of a metal catalyst wherein the metal is selected from the group consisting of Ni, Ir, Pd, Pt, Rh and Ru, particularly Pd or Pt, particularly a heterogeneous metal catalyst on a carrier or a support material.

8. The process according to claim 7, **characterized in that** the carrier is a carbon or an oxide of Si, Al, Ce, Ti or Zr, particularly of Al or Si, preferably carbon.

9. The process according to claim 7 or 8, **characterized in that** the catalyst is Pd on carbon.

10. The process according to anyone of the preceding claims claim 7 to 9, **characterized in that** the surface area of the carrier is in the range of 800 to 1500 m²/g, particularly of 1000 to 1200 m²/g.

11. The process according to claim 7, **characterized in that** the support material is a structured packing element.

12. The process according to anyone of the preceding claims 7-11 **characterized in that** the ratio of the weight of the metal, particularly of Pd or Pt, to the weight of the catalyst is between 1 to 20%, preferably between 4 and 11%, more preferably between 4 and 6% by weight.

13. The process according to anyone of the preceding claims **characterized in that** R¹ = R² = R³ = CH₃.

14. Use of ethylene carbonate and/or propylene carbonate and/or butylene carbonate as a solvent for the reduction of 2,3,5-trimethyl-1,4-benzoquinone to 2,3,5-trimethylhydroquinone.

## Patentansprüche

1. Verfahren zur Herstellung der Verbindung der Formel (I) durch Reduktion der Verbindung der Formel (III) 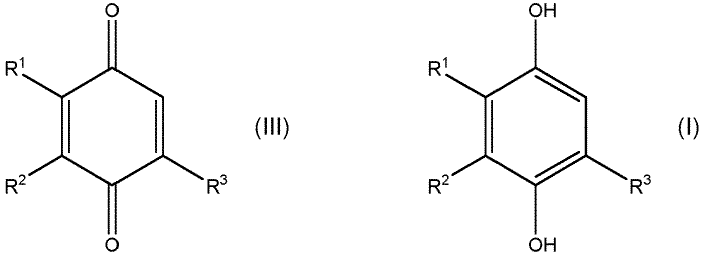
wobei R¹, R² und R³ unabhängig entweder für H oder eine C₁₋₄-Alkylgruppe stehen, mit der Maßgabe, dass mindestens einer der Reste R¹, R² und R³ von H verschieden ist;
**dadurch gekennzeichnet, dass** die Reduktion mit einem Reduktionsmittel in mindestens einem Lösungsmittel der Formel (X) 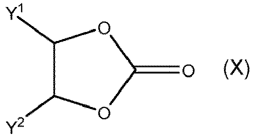
wobei Y¹ oder Y² unabhängig voneinander entweder für H oder eine Methyl- oder Ethylgruppe stehen;
oder in einer zweiphasigen Lösungsmittelmischung, die mindestens ein Lösungsmittel (**A**), bei dem es sich um ein Lösungsmittel der Formel (X) handelt, und mindestens ein Lösungsmittel (**B**), das bei Raumtemperatur nicht mit Lösungsmittel der Formel (X) mischbar ist, in einem Volumenverhältnis (**A**)/(**B**) zwischen 1/50 und 50/1 umfasst, durchgeführt wird.

2. Verfahren nach Anspruch 1, **dadurch gekennzeichnet, dass** Y¹ = Y² = H.

3. Verfahren nach Anspruch 1, **dadurch gekennzeichnet, dass** Y¹ = H und Y² = CH₃.

4. Verfahren nach einem der vorhergehenden Ansprüche, **dadurch gekennzeichnet, dass** die Reduktion in einer Lösungsmittelmischung aus einem Lösungsmittel (**A**) der Formel (X) und einem Lösungsmittel (**B**), bei dem es sich um einen Kohlenwasserstoff, vorzugsweise ein Alkan, weiter bevorzugt ein C₅₋₁₆-Alkan, insbesondere ein C₆₋₈-Alkan, handelt, durchgeführt wird.

5. Verfahren nach einem der vorhergehenden Ansprüche, **dadurch gekennzeichnet, dass** die Reduktion in einer Lösungsmittelmischung aus mindestens zwei Lösungsmitteln der Formel (X), insbesondere einer Mischung aus Ethylencarbonat und Propylencarbonat, durchgeführt wird.

6. Verfahren nach einem der vorhergehenden Ansprüche, **dadurch gekennzeichnet, dass** die Reduktion mit einem Reduktionsmittel durchgeführt wird, bei dem es sich entweder um molekularen Wasserstoff oder ein Transferhydrierungsmittel oder ein Dithionitsalz handelt.

7. Verfahren nach einem der vorhergehenden Ansprüche, **dadurch gekennzeichnet, dass** die Reduktion mit einem Reduktionsmittel, bei dem es sich um molekularen Wasserstoff handelt, in Gegenwart eines Metallkatalysators, wobei das Metall aus der Gruppe bestehend aus Ni, Ir, Pd, Pt, Rh und Ru, insbesondere Pd oder Pt, ausgewählt wird, insbesondere eines heterogenen Metallkatalysators auf einem Träger oder Trägermaterial, durchgeführt wird.

8. Verfahren nach Anspruch 7, **dadurch gekennzeichnet, dass** es sich bei dem Träger um einen Kohlenstoff oder ein Oxid von Si, Al, Ce, Ti oder Zr, insbesondere von Al oder Si, vorzugsweise Kohlenstoff, handelt.

9. Verfahren nach Anspruch 7 oder 8, **dadurch gekennzeichnet, dass** es sich bei dem Katalysator um Pd auf Kohlenstoff handelt.

10. Verfahren nach einem der vorhergehenden Ansprüche 7 bis 9, **dadurch gekennzeichnet, dass** die Oberfläche des Trägers im Bereich von 800 bis 1500 m²/g, insbesondere von 1000 bis 1200 m²/g, liegt.

11. Verfahren nach Anspruch 7, **dadurch gekennzeichnet, dass** es sich bei dem Trägermaterial um ein Packungselement handelt.

12. Verfahren nach einem der vorhergehenden Ansprüche 7-11, **dadurch gekennzeichnet, dass** das Verhältnis von Gewicht des Metalls, insbesondere von Pd oder Pt, zu Gewicht des Katalysators zwischen 1 bis 20 Gew.-%, vorzugsweise zwischen 4 und 11 Gew.-%, weiter bevorzugt zwischen 4 und 6 Gew.-%, liegt.

13. Verfahren nach einem der vorhergehenden Ansprüche, **dadurch gekennzeichnet, dass** R¹ = R² = R³ = CH₃.

14. Verwendung von Ethylencarbonat und/oder Propylencarbonat und/oder Butylencarbonat als Lösungsmittel für die Reduktion von 2,3,5-Trimethyl-1,4-benzochinon zu 2,3,5-Trimethylhydrochinon.

## Revendications

1. Procédé pour la fabrication du composé de la formule (I) par réduction du composé de la formule (III) 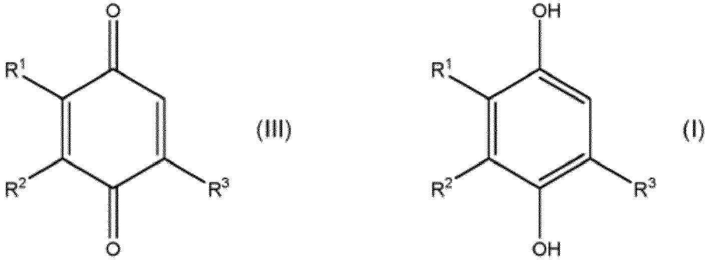
R¹, R² et R³ représentant indépendamment soit H, soit un groupe alkyle en C₁₋₄, à la condition qu'au moins l'un des radicaux R¹, R² et R³ soit différent de H ; **caractérisé en ce que** la réduction est réalisée par un agent de réduction dans au moins un solvant de la formule (X) 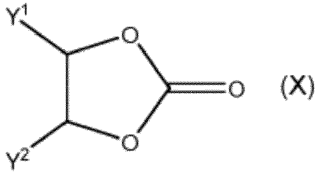 Y¹ et Y² représentant indépendamment l'un de l'autre soit H, soit un groupe méthyle ou éthyle ;
ou dans un mélange de solvants biphasique comprenant au moins un solvant (A) qui est un solvant de la formule (X) et au moins un solvant (B) qui n'est pas miscible avec un solvant de la formule (X) à température ambiante en un rapport volumique de (A)/(B) compris entre 1/50 et 50/1.

2. Procédé selon la revendication 1, **caractérisé en ce que** Y¹ = Y² = H.

3. Procédé selon la revendication 1, **caractérisé en ce que** Y¹ = H et Y² = CH₃.

4. Procédé selon l'une quelconque des revendications précédentes, **caractérisé en ce que** la réduction est réalisée dans un mélange de solvants d'un solvant (A) qui est un solvant de la formule (X) et d'un solvant (B) qui est un hydrocarbure, préférablement un alcane, plus préférablement un alcane en C₅₋₁₆, particulièrement un alcane en C₆₋₈.

5. Procédé selon l'une quelconque des revendications précédentes, **caractérisé en ce que** la réduction est réalisée dans un mélange de solvants d'au moins deux solvants de la formule (X), particulièrement dans un mélange de carbonate d'éthylène et de carbonate de propylène.

6. Procédé selon l'une quelconque des revendications précédentes, **caractérisé en ce que** la réduction est réalisée en utilisant un agent de réduction qui est soit l'hydrogène moléculaire, soit un agent d'hydrogénation par transfert, soit un sel de dithionite.

7. Procédé selon l'une quelconque des revendications précédentes, **caractérisé en ce que** la réduction est réalisée en utilisant un agent de réduction qui est l'hydrogène moléculaire en la présence d'un catalyseur métallique, le métal étant choisi dans le groupe constitué par Ni, Ir, Pd, Pt, Rh et Ru, particulièrement Pd ou Pt, particulièrement un catalyseur métallique hétérogène sur un support ou un matériau de support.

8. Procédé selon la revendication 7, **caractérisé en ce que** le support est un carbone ou un oxyde de Si, Al, Ce, Ti ou Zr, particulièrement de Al ou Si, préférablement un carbone.

9. Procédé selon la revendication 7 ou 8, **caractérisé en ce que** le catalyseur est Pd sur carbone.

10. Procédé selon l'une quelconque des revendications précédentes 7 à 9, **caractérisé en ce que** l'aire de surface du support est dans la plage de 800 à 1 500 m²/g, particulièrement de 1 000 à 1 200 m²/g.

11. Procédé selon la revendication 7, **caractérisé en ce que** le matériau de support est un élément de remplissage structuré.

12. Procédé selon l'une quelconque des revendications 7 à 11, **caractérisé en ce que** le rapport du poids du métal, particulièrement de Pd ou Pt, sur le poids du catalyseur est compris entre 1 et 20 %, préférablement entre 4 et 11 %, plus préférablement entre 4 et 6 % en poids.

13. Procédé selon l'une quelconque des revendications précédentes, **caractérisé en ce que** R¹ = R² = R³ = CH₃.

14. Utilisation de carbonate d'éthylène et/ou de carbonate de propylène et/ou de carbonate de butylène en tant que solvant pour la réduction de 2,3,5-triméthyl-1,4-benzoquinone en 2,3,5-triméthylhydroquinone.
